# EUROPEAN PATENT SPECIFICATION

(11) **EP 2 950 783 B1**
(45) Date of publication and mention of the grant of the patent: **15.05.2019**
(21) Application number: 14704747.6
(22) Date of filing: 03.02.2014
(51) Int. Cl.: A61K 9/10, A61K 47/36, A61K 47/38

(54) **ARTIFICIAL TEARS COMPRISING SODIUM HYALURONATE AND CARBOXYMETHYLCELLULOSE**
KÜNSTLICHE TRÄNEN ENTHALTEND NATRIUMHYALURONAT UND CARBOXYMETHYLCELLULOSE
LARMES ARTIFICIELLES COMPRENANT HYALURONATE SODIUM ET CARBOXYMETHYL CELLULOSE

(30) Priority: 01.02.2013 US 201361759710 P; 14.03.2013 US 201361785857 P
(43) Date of publication of application: 09.12.2015
(60) Divisional application: 19151359.7
(73) Proprietor: ALLERGAN, INC., Irvine, CA 92612 (US)
(72) Inventor: BEARD, Bereth J., Newport Beach, California 92660 (US); BLANDA, Wendy M., Tustin, California 92780 (US); MARSH, David A., Irvine, California 92618 (US); SIMMONS, Peter A., Yorba Linda, California 92886 (US); VEHIGE, Joseph G., Laguna Niguel, California 92677 (US); HAIXIA, Liu, Irvine, California 92612 (US); MATSUMOTO, Steven S., San Clemente, California 92672 (US)
(74) Representative: Hoffmann Eitle
(86) International application number: PCT/US2014/014505
(87) International publication number: WO 2014/121232

(56) References cited:
- EP-A2- 1 992 362
- WO-A1-2009/157596
- WO-A1-2010/047927
- US-A1- 2004 137 079

## Description

### FIELD OF THE INVENTION

Embodiments described herein relate to formulations for and methods of use for eye drop formulations comprising Carboxymethyl cellulose (CMC) and hyaluronic acid (HA) with an improved distribution on the cornea during blinking.

### SUMMARY OF THE RELATED ART

Carboxymethyl cellulose (CMC) and hyaluronic acid (HA) are both compounds that may be used in commercial ophthalmic formulations.

United States Patent 5017229 'Water Insoluble Derivatives of Hyaluronic Acid" discusses a water insoluble biocompatible gel that includes the reaction product of hyaluronic acid, a polyanionic polysaccharide, and an activating agent. CMC is one of the polyanionic polysaccharides claimed. The product claimed is not an eye drop, but a film or a gel.

United States Patent Application 2010/0086512 "Mucomimetic compositions and uses thereof" discloses an eye drop comprising of a cationic antimicrobial agent with a magnesium calcium or magnesium/calcium complex of an anionic polymer such as HA and/or CMC. The patent application claims muco adhesion and maintained efficacy of the antimicrobial agent.

United States Patent 6472379, "Angiogenesis inhibition", discloses a formulation that inhibits angiogenesis and contains HA, CMC, and carbodiimide as a film or as a gel.

In the Asian Journal of Surgery, Volume 33, Issue 1, January 2010, pages 25-30, Yoo Seung Chung et al discuss "Anti-adhesive effect and safety of sodium hyaluronate and sodium carboxymethyl cellulose solution in thyroid surgery". Post-thyroidectomy adhesion was not decreased by using a HA-CMC combination solution.

In the Journal of Cornea and External Disease, Ji Hwan Lee et all discuss "Efficacy of sodiumhyaluronate and carboxymethylcellulse in treating mild to moderate dry eye disease". A clinical study is discussed where patients are given either 0.1 % sodium hyaluronate solution or 0.5% CMC solution. However, the study does not discuss the combination of HA and CMC together.

Genzyme, Inc., produces an anti-adhesion film called Seprafilm. The film is made of HA and CMC.

WO 2009/157596 A1 discloses an ophthalmic composition for the prevention and treatment of ocular diseases, comprising hyaluronic acid or a pharmaceutically acceptable salt thereof, and carboxymethylcellulose or a pharmaceutically acceptable salt thereof.

### BRIEF SUMMARY OF THE INVENTION

The present invention relates to an eye drop formulation comprising carboxymethyl cellulose (CMC) and hyaluronic acid (HA) with an improved distribution on the cornea during blinking. The compositions of the present invention are as defined in the appended claim.

The compositions of the present invention can be administered to a person suffering from Dry Eye Syndrome, and can provide an improved distribution on the cornea during blinking.

The compositions of the present invention may also be used to improve the visual acuity of a person in need thereof. This can be achieved by topically administering the composition of the invention to a person. It can allow an improved distribution on the cornea during blinking. Preferably, said person suffers from Dry Eye Syndrome.

One advantage of CMC is that ocular to systemic ratios are improved with viscous CMC formulations. One advantage of HA is that the shear dependent viscosity of the HA gives an improved distribution on the cornea during blinking.

There is an increase in viscosity which is observed when 0.5% carboxymethyl cellulose (CMC) is mixed with 0.1% hyaluronic Acid (HA). The increase in viscosity is more than additive which is unexpected. These enhanced properties are greater than would be theoretically expected.

### BRIEF DESCRIPTION OF THE DRAWINGS

**Figure 1** describes the viscosity curves determined for CMC in PBS, HA in PBS, CMC with HA measured and CMC with HA expected.
**Figure 2** describes the Brookfield viscosity at 60 RPM determined for CMC in PBS, HA in PBS, CMC with HA measured and CMC with HA expected.
**Figures 3A-I** describe clinical study results and scoring for tested formulations in various categories pursuant to a study described in greater detail below during the length of the study.
**Figure 4** describes the change from baseline in tear breakup time for tested formulations pursuant to a study described in greater detail below during the length of the study.
**Figure 5** illustrates the change in baseline pursuant to a study described in greater detail below for combined corneal and conjunctival staining in tested formulations during the length of the study.
**Figure 6** illustrates the change in baseline pursuant to a study described in greater detail below for combined corneal and conjunctival staining in tested formulations during the length of the study for a subgroup showing clinically-important staining.
**Figure 7** illustrates the change in baseline pursuant to a study described in greater detail below for corneal staining in tested formulations during the length of the study for a subgroup showing clinically-important staining.
**Figure 8** illustrates the change in baseline pursuant to a study described in greater detail below for corneal staining in tested formulations during the length of the study.
**Figure 9** illustrates the change in ocular burning and stinging during the length of a study described in greater detail below.
**Figure 10** illustrates the change in ocular dryness during the length of a study described in greater detail below.
**Figure 11** illustrates the change in lid wiper epitheliopathy during the length of a study described in greater detail below.

### DETAILED DESCRIPTION OF THE INVENTION

In one aspect of the invention, there is provided an eye drop formulation as defined in the appended claim 1.

One advantage of CMC is that ocular to systemic ratios are improved with viscous CMC
formulations. One advantage of HA is that the shear dependent viscosity of the HA gives an improved distribution on the cornea during blinking.

There is an increase in viscosity which is observed when about 0.1% to about 1.0% Carboxymethyl cellulose (CMC) is mixed with about 0.05% to about 0.15% Hyaluronic Acid (HA). There is an increase in viscosity which is observed when about 0.5% Carboxymethyl cellulose (CMC) is mixed with about 0.1% Hyaluronic Acid (HA). There is an increase in viscosity which is observed when 0.5% Carboxymethyl cellulose (CMC) is mixed with a 0.1% Hyaluronic Acid (HA). The increase in viscosity is more than additive which is unexpected. Therefore, we claim the discovery of a new eye drop formulation which has enhanced properties in comparison to formulations with single components. These enhanced properties are greater than would be theoretically expected.

The following Table lists some examples of preferred formulations.

**Table 1: Formulations**

| **Ingredient** | **Formulation 1: Concentration, % (w/v)** | **Formulation 2: Concentration, % (w/v)*** | **Formulation 3: Concentration, % (w/v)*** | **Role** |
|---|---|---|---|---|
| Carboxymethyl cellulose sodium | 0.5 % w/v | 0.5 % w/v | 0.5 % w/v | Active |
| Glycerin | 0.9 % w/v | 0.9 % w/v | 0.9 % w/v | Active |
| Sodium hyaluronate | 0.1 % w/v | 0.15 % w/v | 0.10 % w/v | Viscosity adj. |
| Purite® | 0.01 % w/v | 0.01 % w/v | 0.01 % w/v | Preservative |
| Boric acid | 0.7 % w/v | 0.7 % w/v | 0.7 % w/v | Buffering agent |
| Sodium borate decahydrate | 0.2 % w/v | 0.2 % w/v | 0.2 % w/v | Buffering agent |
| Sodium citrate dihydrate | 0.1 % w/v | 0.1 % w/v | 0.1 % w/v | Buffering agent |
| Potassium chloride | 0.14 % w/v | 0.14 % w/v | 0.14 % w/v | Excipient |
| Calcium chloride dihydrate | 0.006 % w/v | 0.006 % w/v | 0.006 % w/v | Excipient |
| Magnesium chloride hexahydrate | 0.006 % w/v | 0.006 % w/v | 0.006 % w/v | Excipient |
| Erythritol | 0.5 % w/v | 0.5 % w/v | 0.25 % w/v | Excipient |
| Levocarnitine | -- | -- | 0.25 % w/v | Excipient |
| Sodium hydroxide | 7.2 pH | 7.2 pH | 7.2 pH | pH Adjust |
| Hydrochloric acid | 7.2 pH | 7.2 pH | 7.2 pH | pH Adjust |
| Water for injection /purified water | 100 % w/v | 100 % w/v | 100 % w/v | QS Adjust |

| | | | | |
|---|---|---|---|---|
| * = not part of present invention | | | | |

The carboxymethyl cellulose used in embodiments described herein is preferably of any grade that can be formulated for topical use. The carboxymethyl cellulose is sodium carboxymethyl cellulose. More preferably, the carboxymethyl cellulose is sodium carboxymethyl cellulose with a low viscosity.

The HA used in embodiments described herein is preferably of any grade that can be formulated for topical use. Preferably, the hyaluronic acid is in the form of sodium hyaluronate. In some embodiments, the hyaluronic acid has an intrinsic viscosity of about 0.5 m³/kg to about 4.0 m³/kg, more preferably about 1.1 m³/kg to about 2.0 m³/kg, or also more preferably about 2.5 m³/kg to about 4.0 m³/kg. Some embodiments may have an intrinsic viscosity of about 2.2 m³/kg to about 2.6 m³/kg, and some embodiments may have an intrinsic viscosity of about 1.1 m³/kg to about 3.0 m³/kg.

This formulation demonstrates a marked drop in viscosity upon application of a shear force; the use of a wetting drop with a high shear drop in viscosity suggests that, when the formulation is dropped in the eye, the eyelid will produce sufficient shear-force to produce a low-viscosity, comfortable, eye drop.

But, once in the cul-de-sac (i.e., an eye corner) where there is no shear, the formulation should rapidly become more viscous. Having this "reservoir" of viscous polymers will maintain a sustained level of polymers in the tear fluid. The polymers in the tear fluid will limit the evaporation of tears and, in this way, counter dry eye. Concomitantly, the sheer force of the eyelid should prevent the polymers from becoming too viscous and blurring vision.

In addition, the viscous material in the cul-de-sac (e.g., in the puncta and in the canaliculus) slows the drain of tears from the eye and therefore, has a second action to treat dry eye by building tear fluid up in front of the eye.

In some embodiments, compositions and methods directed to eye drop compositions include an alpha-hydroxyl acid ("AHA") which is sodium lactate.

It has been found that AHA-containing compositions and formulations may benefit from the inclusion of an AHA, as cells-such as cells already damaged by dry eye and other ocular conditions-can be gently and selectively exfoliated by an AHA. Accordingly, some embodiments may comprise the administration of an AHA-containing formulation that also comprises compatible solutes. Without wishing to be bound by theory, it is believed that such formulations may protect cells (e.g., ocular and conjunctival cells) from stress and facilitate cell renewal. This may increase the overall ocular health of the eye to which the formulation is applied.

A normal ocular surface is covered with non-keratinized corneal and conjunctival epithelial cells. The non-keratinized superficial epithelial cells produce bound mucins (glycocalyx) that coat the ocular surface to create a hydrophilic surface that stabilizes the tear film. Goblet cells are interspersed between conjunctival epithelial cells, and secret soluble unbound mucins that stabilize the tear film by reducing surface tension, as well as lubricate and protect the ocular surface. For the special structure and function of ocular surface, it is most preferable to keep the epithelial cells non-keratinized to maintain its health and integrity.

However, in an adverse condition like dry eye, the ocular surface integrity may be disrupted due to the stress of hyperosmolarity and/or desiccation. Again, without wishing to be bound by theory, some studies have shown that hyperosmotic stress can activate the mitogen-activated protein kinase signaling pathway, which further leads to squamous metaplasia, or "cornification". Cornification disrupts the corneal epithelial barrier function. The cornified epithelial cells may then lose their glycocalyx coverage and become poorly hydrated, thereby destabilizing the tear film and exacerbating ocular desiccation. Some research has demonstrated that the cornified conjunctival epithelial cells can entrap the conjunctival goblet cells, blocking their mucus secretion, and further degrading the tear quality and stability.

Accordingly, it would be advantageous to provide an AHA-containing formulation that protects ocular epithelial cells from stress and/or cornification. Also, it would be advantageous to provide an AHA-containing formulation that facilitates the shedding of cornified cells, thereby improving the renewal of the ocular surface.

The AHA-containing formulation comprises one or more solute components which are levocarnitine, betaine, glycerin, and erythritol. Some studies have shown that these small molecules may protect cells from hyperosmotic stress. Again, without wishing to be bound by theory, it is believed that these solute components may enter and accumulate in cells, thereby balancing the osmotic difference with the extracellular fluid. Some studies on the cultured corneal epithelial cells have found that these and similar solutes significantly lowered the levels of MAPK activation in response to hyperosmotic stress. As it is believed that cornification may be mediated by MAPK pathway, compatible solutes may prevent cell cornification, as well as inflammation and other damage.

AHA, including lactic acid and glycolic acid, has been widely used in skin care as a gentle exfoliator at low concentration. AHA is believed to selectively affect epithelial keratinization, thereby diminishing the cellular cohesion between corneocytes at the lowest levels of the stratum corneum. This action promotes exfoliation of the outermost layer of the skin. Especially when used in high concentration and low pH, AHA can produce a rapid loss of skin cells, as seen in a "chemical peel." However, the exfoliating effect of AHA is not solely attributable to its low pH, as many AHA containing skin care products have a pH of near neutral with a gentle but efficient exfoliating effect.

Accordingly, in one embodiment, a preferred composition comprises a sodium lactate buffer. This composition may be beneficial in promoting the shedding of cornified epithelial cells so as to maintain ocular surface integrity. The removal of the cornified cells may also eliminate the formation of dry spots on the ocular surface and enhance tear film distribution over the ocular surface. In addition, removal of cornified epithelia may open up the entrapped goblet cells and help the recovery of goblet cell function to allow better delivery of mucins to the ocular surface. The combination of the two effects may further be beneficial in stabilizing the tear film and in protecting the ocular surface. The lactate buffer may also be more biocompatible with other commonly-used buffers, as lactate acid is a by-product of glucose metabolism that naturally exists in the tears. Studies have shown that lactate may participate in wound healing, stimulating collagen and hyaluronan synthesis. In addition, as a small solute, lactate may also serve as an intra-cellular osmolyte that protects the ocular surface from hyperosmotic stress by a mechanism similar to or synergistic with the osmoprotectant compatible solutes. The potential osmoprotection of lactate may help further reduce cornification.

A preferred AHA-containing formulation is set out in Table 2 below.

**Table 2: Formulations**

| **Ingredient** | **Formulation 4: Concentration, % (w/v)** | **Role** |
|---|---|---|
| Carboxymethylcellulose sodium | 0.5 % w/v | Active |
| Glycerin | 1.0 % w/v | Active |
| Sodium hyaluronate | 0.1 % w/v | Viscosity adj. |
| Sodium lactate | 0.3% w/v | Buffering agent |
| Potassium chloride | 0.14 % w/v | Excipient |
| Calcium chloride dihydrate | 0.006 % w/v | Excipient |
| Magnesium chloride hexahydrate | 0.006 % w/v | Excipient |
| Erythritol | 0.5 % w/v | Excipient |
| Levocarnitine | 0.25% w/v | Excipient |
| Sodium hydroxide | 6.2-7.0 pH | pH Adjust |
| Hydrochloric acid | 6.2-7.0 pH | pH Adjust |
| Water for injection /purified water | 100 % w/v | QS Adjust |

The carboxymethyl cellulose used in embodiments described herein is preferably of any grade that can be formulated for topical use. The carboxymethyl cellulose is sodium carboxymethyl cellulose. More preferably, the carboxymethyl cellulose is sodium carboxymethyl cellulose with a low viscosity.

The HA used in embodiments described herein is preferably of any grade that can be formulated for topical use. Preferably, the hyaluronic acid is in the form of sodium hyaluronate. In some embodiments, the hyaluronic acid has an intrinsic viscosity of about 0.5 m³/kg to about 4.0 m³/kg, more preferably about 0.9 m³/kg to about 3.0 m³/kg. In some preferred embodiments, the hyaluronic acid has an average molecular weight from about 2.0 to about 2.6 million Daltons. In some other preferred embodiments, the hyaluronic acid has an intrinsic viscosity from about 1.1 m³/kg to about 2.0 m³/kg. In some other preferred embodiments, the hyaluronic acid has an average molecular weight from about 0.5 to about 1.2 million Daltons.

### Example 1

In one test, shown below, 0.5% CMC and 0.1 % HA solutions were tested for viscosity in comparison to a solution that contained both 0.5% CMC and 0.1 % HA (Formulation 1 in the table above). At several different frequencies tested, the viscosity of the combination formulation was greater than the predicted value (calculated by addition of the values for the individual formulations). This shows that the desired viscosity can be obtained by using less CMC and/or HA than one would predict based, if a combination is utilized instead of a single polymer.

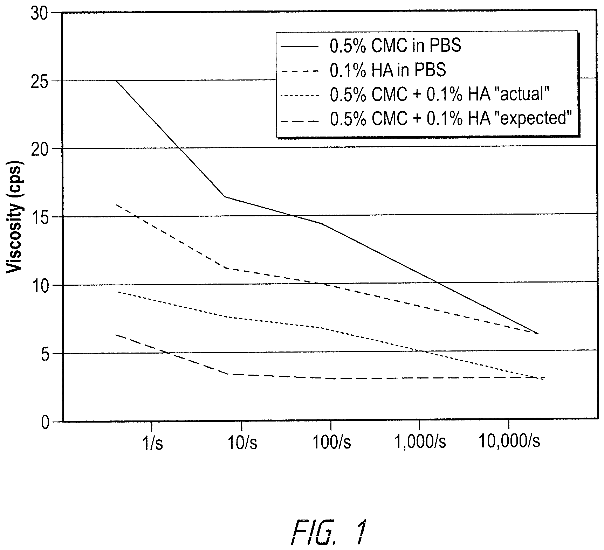

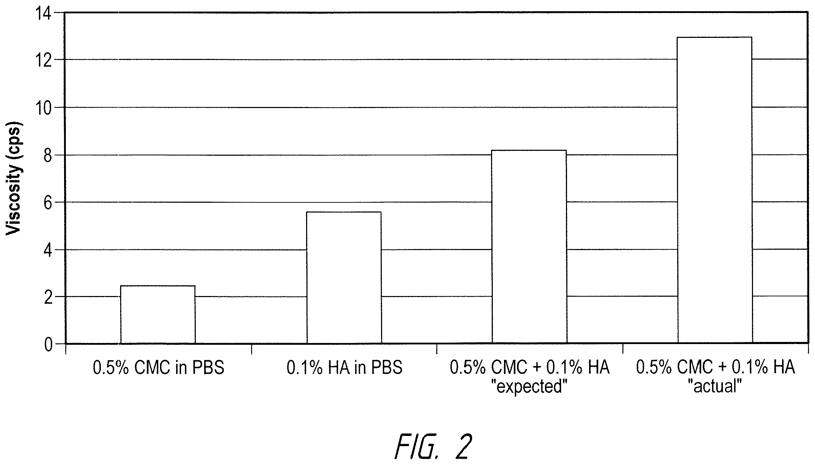

As stated above, the shear thinning characteristic is a desirable characteristic of HA formulations. However, HA is quite expensive in comparison to CMC. In the example where 0.5% CMC and 0.1 % HA solutions were tested for viscosity in comparison to a solution that contained both 0.5% CMC and 0.1 % HA, shear thinning can be quantified by taking the ratio of the viscosity, in centipoise (cps), at *1*/*s* to the viscosity at 10/s. These results are summarized in Table 3 below, as well as Figure 1. Figure 2 illustrates the viscosity in cps at 60rpm.

**Table 3: Shear thinning**

| | Frequency (1/s) | | | | | | |
|---|---|---|---|---|---|---|---|
| Formulation | 1 | 10 | 100 | 1000 | 10000 | 30 rpm | 60 rpm |
| 0.5% CMC in PBS | 6.4 | 3.6 | 3.2 | 3.2 | 3.2 | 2.5 | 2.5 |
| 0.1 % HA in PBS | 9.5 | 7.7 | 6.7 | 4.8 | 3.2 | 5.7 | 5.7 |
| Predicted 0.5% CMC +0.1% HA | 15.9 | 11.3 | 9.9 | 8 | 6.2 | 8.2 | 8.2 |
| Actual 0.5% CMC + 0.1% HA | 25 | 16.5 | 14.2 | 10.2 | 6.2 | 13.5 | 13.1 |

Although there is more CMC than HA in the formulation, the shear thinning ratio of the combination is high - close to that of the HA alone. This shows that a CMC formulation can be made to shear thin with the addition of HA.

At this time, it is not known if the 0.5% CMC to 0.1 % HA is the optimal ratio of these polymers. An even greater gain in viscosity and enhanced shear thinning might be realized by a slightly different combination of CMC and HA.

Without wishing to be bound by theory, it is possible that the unexpected increase in viscosity demonstrates that the polymers have an unexpected positive interaction. This interaction is unlikely to be charge-charge interaction since both sodium hyaluronate and sodium carboxymethyl cellulose are slightly negatively charged when in solution. The interaction is likely chain entanglement, which can have a positive clinical benefit. It is theorized, clinically, that CMC may have more binding force to corneal cells while HA is a better lubricant but has less ocular surface retention. In combination, the CMC assists in HA retention, via entanglement, to the cell membrane.

The HA-CMC-cornea attraction gives a better, more durable lubricant system. In addition, since both polymers have been shown to contribute to cell migration *in vitro,* there could be a combined enhanced benefit *in vivo.*

### Example 2

Testing was performed on human volunteers, using two CMC-HA formulations. The first formulation used a 0.5% CMC/0.1% HA formulation ("EDNP-1") as set forth above as Formulation 1 in Table 1. The second formulation used a 0.5% CMC/0.15% HA formulation ("EDNP-2") as set forth above as Formulation 2 in Table 1. The two Formulations were compared to an existing product known to be effective in treating dry eye-Refresh® Tears with 0.5% CMC, salts, and Purite® ("Refresh"). It is important to note that the Refresh product used in testing does not, strictly speaking, represent a control with little therapeutic activity (e.g., pure saline). Rather, the Refresh product represents an established, successful product that is known to be efficacious in treating dry eye.

Approximately 100 individuals were in each treatment group, with dosing done as needed, and at least twice daily, for 90 days. The primary endpoint was a change in the Ocular Surface Disease Index (OSDI) at day 90 of the testing. The OSDI is a 12-question standardized test that surveys patients on the existence and severity of various dry-eye related attributes, including light sensitivity, perceived eye grittiness, vision blurring, and eye comfort at various times and when performing various activities.

The study population consisted of current eye drop users with mild to severe symptoms and signs of dry eye, so as to obtain a generally heterogeneous population that would provide a good indication of the efficacy of the two tested Formulations. The population therefore presented with an OSDI between 18 and 65, a tear breakup time (TBUT) of less than 10 seconds, and presenting some corneal and/or conjunctival surface staining. Patients showing especially severe dry eye were excluded.

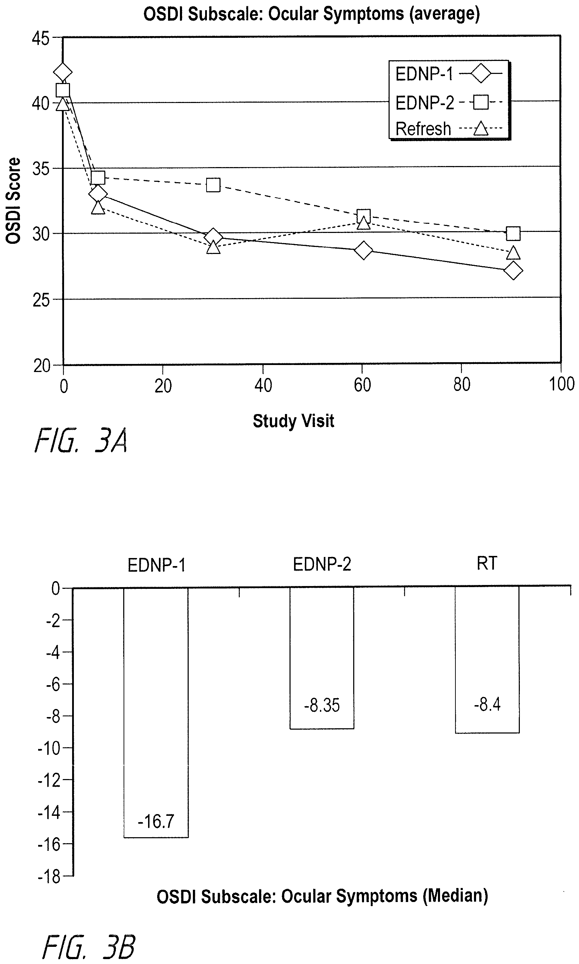

Figures 3A-F depict the average scoring in specific OSDI sub-categories for the tested formulations. In Figure 3A, OSDI ocular symptoms for EDNP-1 appeared to be improved over Formulations 2 and the Refresh product. This is evidenced by the lower average OSDI score (or improvement from baseline) at the end of the 90-day period. Figure 3B, showing the median OSDI ocular symptom score, also shows that EDNP-1 has a greater median improvement compared to the other Formulations.

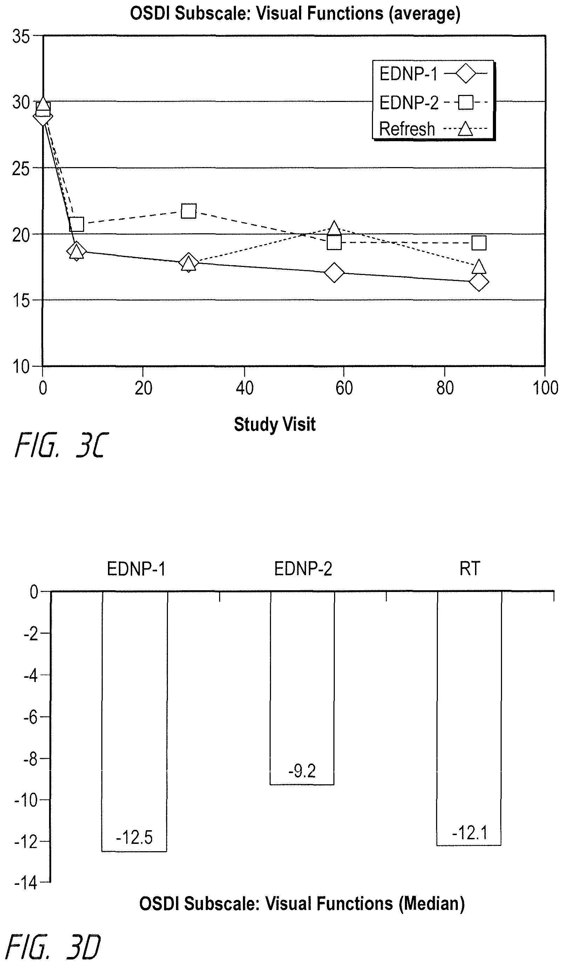

Figure 3C shows that the average OSDI visual function score for EDNP-1 also demonstrates the greatest level of improvement compared to EDNP-2 and Refresh by the end of the 90-day period. In Figure 3D, the median OSDI visual function score shows that EDNP-1 is slightly better than Refresh.

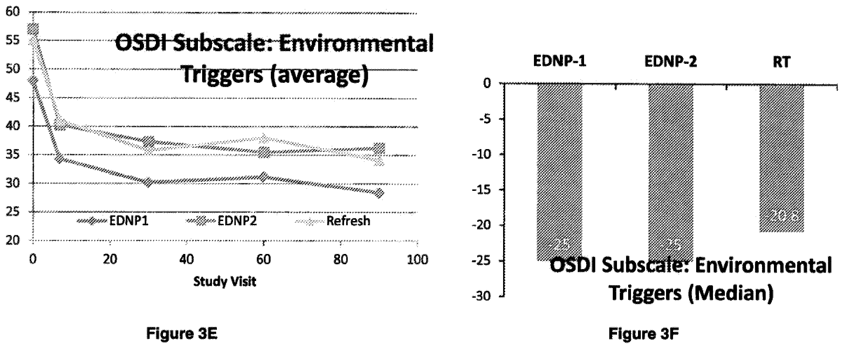

Figure 3E illustrates that the average OSDI score for environmental triggers also shows the most improvement for EDNP-1 compared to EDNP-2 and Refresh by the end of the 90-day period. The median score illustrated in Figure 3F shows that EDNP-1 and -2 are both superior to Refresh.

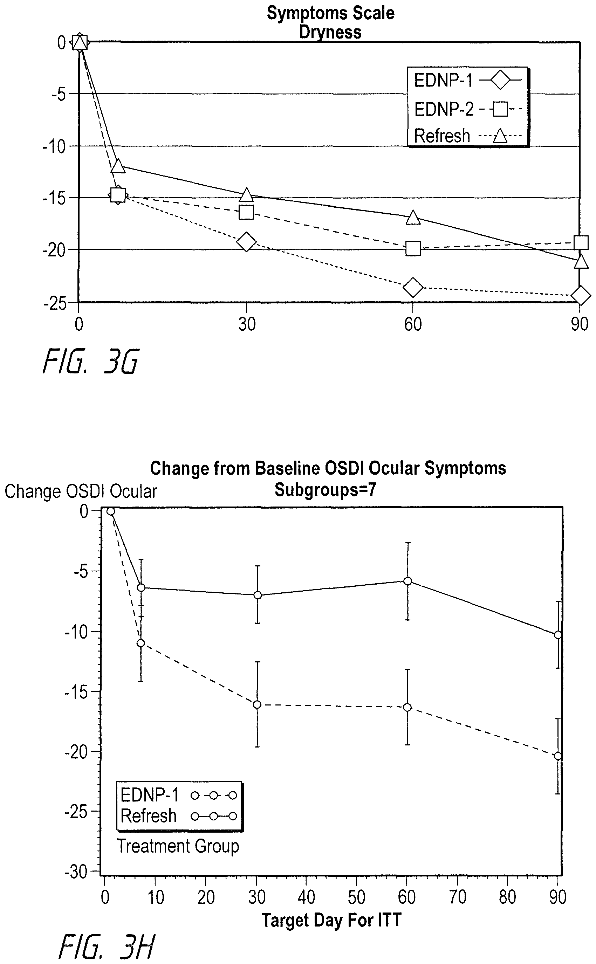

Figure 3G looks specifically to the average dryness symptom score for perceived ocular dryness during the length of the study. Here, while all compositions registered some improvement over the baseline, EDNP-1 showed the greatest improvement over the study duration.

Figure 3H illustrates the results of administering EDNP-1 as compared to Refresh in a study subgroup suffering from severe dry eye, as evidenced by a combined corneal and conjunctival staining score greater than or equal to 15 (the relevance of staining to the severity of dry eye is discussed in greater detail below). Here, EDNP-1 performed better than Refresh at all followup visits from day 7 to day 90, as shown by the reduction in the OSDI score from baseline.

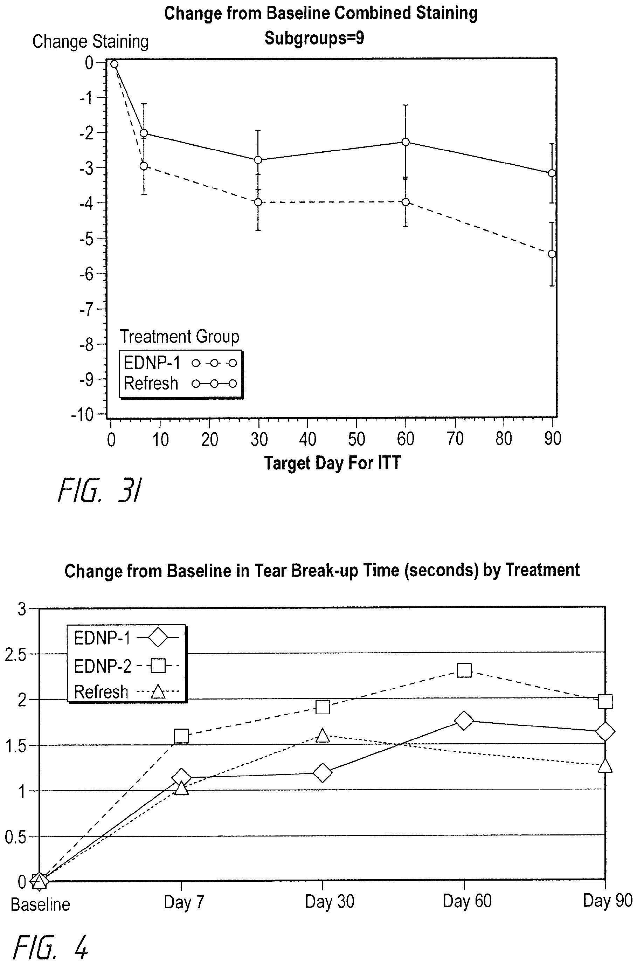

Figure 3I illustrates the change in staining score (again, discussed in greater detail below) for a study subgroup suffering from severe dry eye, based on a VAS eye dryness symptom scale greater than or equal to 66. The VAS eye dryness symptom scale rates the overall severity for ocular dryness in a subject over the course of a week, where a score of zero indicates no ocular dryness, to a maximum possible score of 100, indicating maximal dryness. The VAS eye dryness scale measures the single symptom of ocular dryness, in contrast with the OSDI score being a comprehensive assessment of dry eye symptoms including ocular discomfort, environmentally-triggered, and vision-related symptoms. Here, the EDNP-1 formulation performed better than Refresh at reducing corneal and conjunctival staining for the duration of the study.

Figure 4 is a graph showing the average change in tear break up time, in seconds, from the beginning of the study through 90 days. Here, EDNP-2 showed a greater average change in tear break up time, compared to EDNP-1 and Refresh.

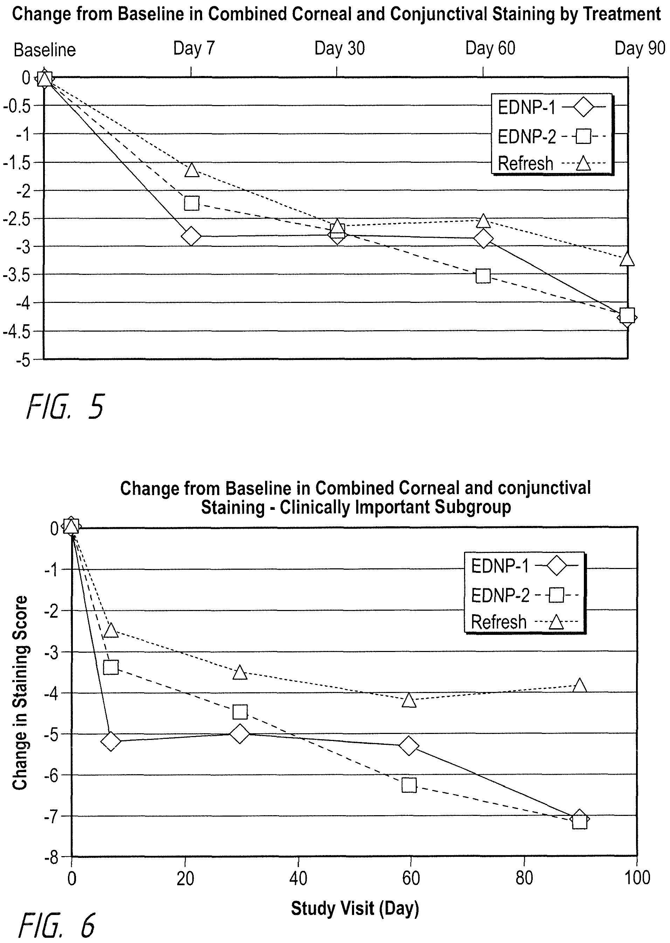

Figure 5 shows the average change during the study from baseline of combined corneal and conjunctival staining in the entire treatment group. Generally, corneal and conjunctival staining is determined by administering a dye to the eye which is capable of staining the exposed ocular surfaces, such as fluorescein, rose bengal, and lissamine green. Staining is believed to indicate areas of the eye which have been damaged as a result of dry eye, and it has been postulated that the dyes stain ocular epithelial surfaces that are lacking a protective mucin protein coat or that otherwise present exposed, unprotected epithelial cell surfaces. Ocular staining can be scored based on the location and amount of staining on different sections and regions of the eye. For example, the cornea may be subdivided into several sections, and the amount of staining quantified and assigned a score. Similar scoring may be applied to the conjunctiva. Examples of such methodologies may be found, for example, in Foulks, "Challenges and Pitfalls in Clinical Trials of Treatments for Dry Eye," Ocular Surface, V. 1, N. 1, pp. 20-30 (2003); and Baudouin, et al, "Randomized, phase III study comparing osmoprotective carboxymethylcellulose with sodium hyaluronate in dry eye disease," Eur J Ophthalmol, 2012; 22(5):751-761.

In the present study, a modified NEI Grid was used with a total of 11 zones distributed throughout the eye. Scoring was assigned using a similar rationale to the Baudouin study cited above, where different ocular zones were scored in relation to the extent of staining, which therefore indicated the severity of the dry eye. Here, EDNP-1 and -2 were better than Refresh in reducing ocular staining, and in particular by the end of the study.

Turning now to Figure 6, average corneal and conjunctival staining scores were tracked during the length of the study for the clinically-important staining subgroup, and compared to the staining score at the beginning of the study. A clinically important staining score was set to be greater than or equal to 15, and the clinically-important staining subgroup was defined as such. This score was used as an indication of patients suffering from more severe dry eye, and the scoring indicates that at least a third of the 11 ocular zones would have a staining score greater than 1. Here, both EDNP-1 and -2 were significantly better than Refresh at reducing corneal and conjunctival staining in a study population with severe dry eye, as shown by the improvement (reduction) in staining scores.

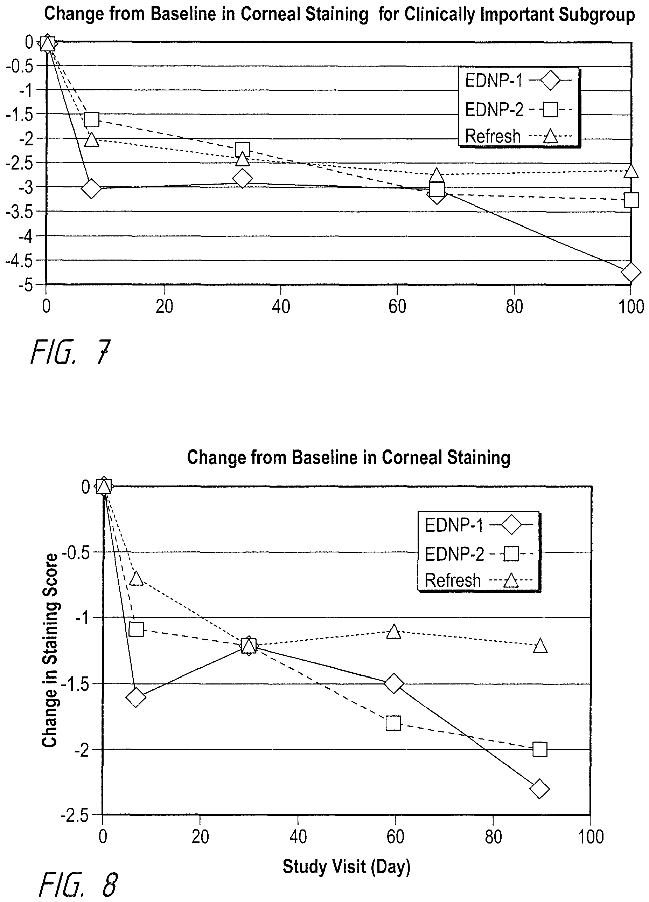

Focusing on the extent of the corneal staining in the clinically important subgroup, Figure 7 shows the average improvement from baseline in this group during the length of the study. Specifically, by the endpoint of the study, EDNP-1 was found to be far superior to the other two formulations in reducing the extent of corneal staining, and as such dry eye, in the group demonstrating clinically-important ocular staining. EDNP-2 was also marginally better than Refresh.

Figure 8 shows a graph of the average corneal staining score change in all subjects, tracked during the length of the entire study. Here, both EDNP-1 and -2 were better than Refresh formulation, with EDNP-1 showing the greatest reduction in corneal staining from baseline as compared to the other tested substances. As such, the tested formulations provide a therapeutic benefit to the majority of study participants and not only the group showing clinically-important ocular staining.

### Example 3

Another clinical test was performed on human volunteers. Here, 365 contact lens users were randomized in a 2:1 treatment allocation with stratification by 6 types of hydrogel, silicon hydrogel, and rigid gas-permeable contact lenses. They were instructed to instill 1 to 2 drops of the studied eye drops in each eye a minimum of 4 times per day for 90 days. One of these uses may be to prepare the contact lens for wear by placing 1 to 2 drops on the inner surface of the contact lens prior to insertion. In this study, Formulation 1 was compared to the Refresh Tears® ("Refresh") eye drops previously described.

The contact lens users were randomized into the study at fifteen different sites in the United States, with 350 subjects (95.9%) completing the study. There were no significant differences between treatment groups in regard to age, sex, or race, or in regard to the number of treatment-related discontinuations.

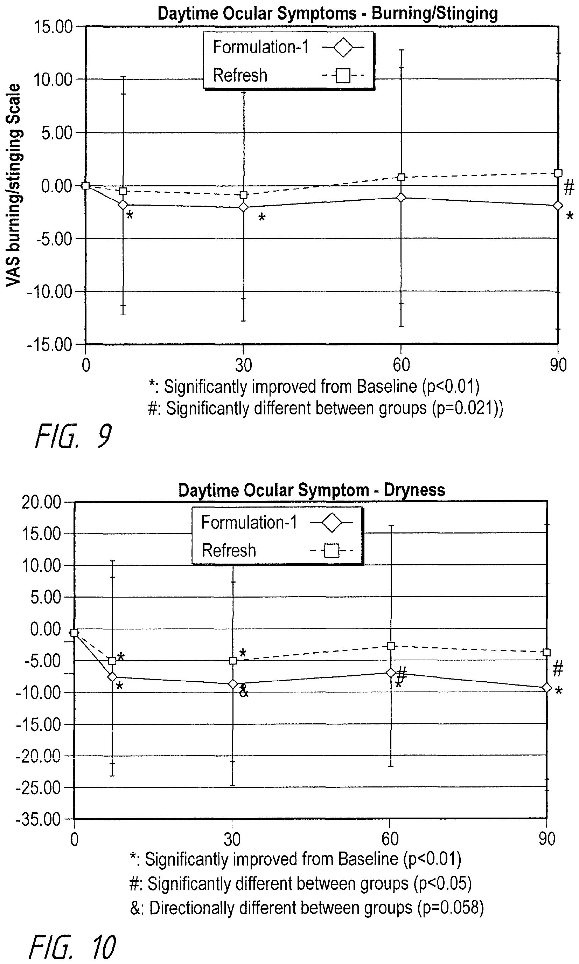

As shown in Figure 9, ocular symptoms of burning and stinging throughout the day were assessed on a VAS burning/stinging scale over a total period of 90 days. A statistically-significant improvement from baseline in users of Formulation 1 in the reduction of symptoms of burning and stinging was shown at 7, 30, and 90 days. Also, by the end of the 90 day period, there was a statistically significant difference and improvement in the reduction of burning and stinging symptoms by Formulation 1 as compared to Refresh.

With reference now to Figure 10, the treatment of the ocular symptom of dryness after use of Formulation 1 was compared to Refresh. Here, Formulation 1 showed a significant improvement from baseline dryness at 7, 30, 60, and 90 days from administration. Further, Formulation 1 showed a statistically significant improvement in the reduction of ocular dryness as compared to Refresh by at least days 60 and 90. This shows that Formulation 1 provides superior long-term reduction in ocular dryness.

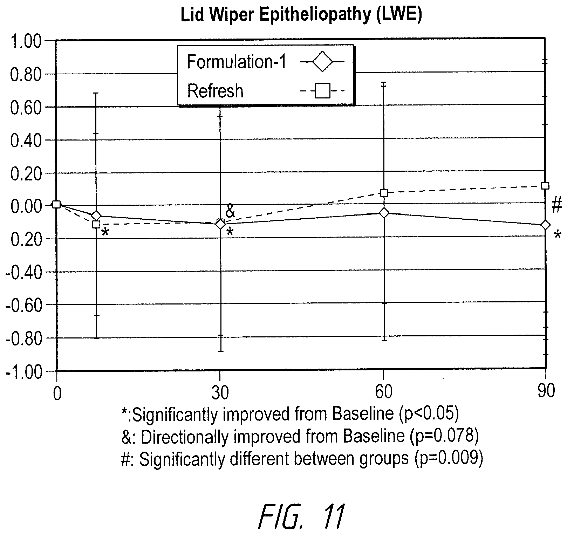

Figure 11 compares the progression of lid wiper epitheliopathy (LWE) between Formulation 1 and Refresh. LWE is a disorder of the marginal conjunctiva of the upper eyelid with dry eye symptoms. LWE may be related to mechanical forces during blinking resulting in inflammation of the ocular surface. LWE was assessed in the study with lissamine green with Korb's Grading (Korb et al, 2010). The upper eyelid was everted with great care to avoid contact with the region of the lid wiper. White light of low-to-moderate intensity with diffuser is used for LWE observation, and LWE was graded from 0 (< 2 mm) to 3 (> 10 mm) based on the horizontal length involved and from 0 (< 25%) to 3 (> 75%) based on the average sagittal height involved. The individual grades for these 2 findings were averaged for a final lissamine green staining grade for the lid wiper of each eye. Here, Formulation 1 showed improvement in reducing LWE from baseline, and was significantly better than Refresh at the end of the 90 day period.

Accordingly, this study demonstrates that Formulation 1 is superior to Refresh at ameliorating and treating dry eye symptoms.

## Claims

1. A composition useful as an artificial tear, wherein the composition is as defined in the table below:
| **Ingredient** | **Formulation 1: Concentration, % (w/v)** | **Formulation 4: Concentration, % (w/v)** |
|---|---|---|
| Carboxymethyl cellulose sodium | 0.5 % w/v | 0.5 % w/v |
| Glycerin | 0.9 % w/v | 1.0 % w/v |
| Sodium hyaluronate | 0.1 % w/v | 0.1 % w/v |
| Purite | 0.01 % w/v | - |
| Boric acid | 0.7 % w/v | - |
| Sodium lactate | - | 0.3% w/v |
| Sodium borate decahydrate | 0.2 % w/v | - |
| Sodium citrate dihydrate | 0.1 % w/v | - |
| Potassium chloride | 0.14 % w/v | 0.14 % w/v |
| Calcium chloride dihydrate | 0.006 % w/v | 0.006 % w/v |
| Magnesium chloride hexahydrate | 0.006 % w/v | 0.006 % w/v |
| Erythritol | 0.5 % w/v | 0.5 % w/v |
| Levocarnitine | - | 0.25% w/v |
| NaOH/HCl for pH adjustment | 7.2 pH | 6.2-7.0 pH |
| Water for injection /purified water | 100 % w/v | 100% w/v |

## Patentansprüche

1. Zusammensetzung, die als künstliche Träne verwendbar ist, wobei die Zusammensetzung wie in der nachstehenden Tabelle definiert ist:
| **Bestandteil** | **Formulierung 1: Konzentration % (G/V)** | **Formulierung 4: Konzentration % (G/V)** |
|---|---|---|
| Natrium-carboxymethylcellulose | 0,5 % G/V | 0,5 % G/V |
| Glycerin | 0,9 % G/V | 1,0 % G/V |
| Natriumhyaluronat | 0,1 % G/V | 0,1 % G/V |
| Purit | 0,01 % G/V | --- |
| Borsäure | 0,7 % G/V | --- |
| Natriumlactat | --- | 0,3 % G/V |
| Natriumboratdecahydrat | 0,2 % G/V | --- |
| Natriumcitratdihydrat | 0,1 % G/V | --- |
| Kaliumchlorid | 0,14 % G/V | 0,14 % G/V |
| Calciumchloriddihydrat | 0,006 % G/V | 0,006 % G/V |
| Magnesiumchloridhexahydrat | 0,006 % G/V | 0,006 % G/V |
| Erythritol | 0,5 % G/V | 0,5 % G/V |
| Levocarnitin | --- | 0,25 % G/V |
| NaOH/HCl zur pH-Einstellung | 7,2 pH | 6,2-7,0 pH |
| Wasser für Injektionszwecke / gereinigtes Wasser | 100 % G/V | 100 % G/V |

## Revendications

1. Composition utile en tant que larme artificielle, dans laquelle la composition est telle que définie dans le tableau ci-dessous :
| **Ingrédient** | **Formulation 1 : Concentration, % (poids/volume)** | **Formulation 4 : Concentration, % (poids/volume)** |
|---|---|---|
| Carboxyméthylcellulose sodique | 0,5 % en poids/volume | 0,5 % en poids/volume |
| Glycérine | 0,9 % en poids/volume | 1,0 % en poids/volume |
| Hyaluronate de sodium | 0,1 % en poids/volume | 0,1 % en poids/volume |
| Purite | 0,01 % en poids/volume | - |
| Acide borique | 0,7 % en poids/volume | - |
| Lactate de sodium | - | 0,3 % en poids/volume |
| Borate de sodium décahydraté | 0,2 % en poids/volume | - |
| Citrate de sodium dihydraté | 0,1 % en poids/volume | - |
| Chlorure de potassium | 0,14 % en poids/volume | 0,14 % en poids/volume |
| Chlorure de calcium dihydraté | 0,006 % en poids/volume | 0,006 % en poids/volume |
| Chlorure de magnésium hexahydraté | 0,006 % en poids/volume | 0,006 % en poids/volume |
| Érythritol | 0,5 % en poids/volume | 0,5 % en poids/volume |
| Lévocarnitine | - | 0,25 % en poids/volume |
| NaOH/HCl pour ajustement de pH | pH 7,2 | pH 6,2-7,0 |
| Eau pour injection /eau purifiée | 100 % en poids/volume | 100 % en poids/volume |
